(19) 

Europäisches
Patentamt

European
Patent Office

Office européen
des brevets

(11) **EP 3 293 265 A1**

(12) **EUROPEAN PATENT APPLICATION**
published in accordance with Art. 153(4) EPC

(43) Date of publication:
**14.03.2018 Bulletin 2018/11**

(21) Application number: **16789638.0**

(22) Date of filing: **04.05.2016**

(51) Int Cl.:
*C12N 15/85* (2006.01)  *C12N 15/62* (2006.01)
*C12N 5/078* (2010.01)  *C12N 5/09* (2010.01)
*C07K 16/28* (2006.01)  *C07K 19/00* (2006.01)
*A61K 47/48* (0000.00)

(86) International application number:
**PCT/KR2016/004750**

(87) International publication number:
**WO 2016/178532 (10.11.2016 Gazette 2016/45)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **04.05.2015 KR 20150062604
27.08.2015 KR 20150120934**

(71) Applicant: **Cellex Life Sciences, Incorporated
Daejeon 34051 (KR)**

(72) Inventors:
• **CHOI, Chulhee
Daejeon 34112 (KR)**

• **YIM, Nambin
Daejeon 34141 (KR)**
• **HEO, Won Do
Daejeon 34125 (KR)**
• **RYU, Seung-Wook
Daejeon 34907 (KR)**
• **CHOI, Hojun
Daejeon 34141 (KR)**
• **CHOI, Kyungsun
Daejeon 34075 (KR)**

(74) Representative: **V.O.
P.O. Box 87930
Carnegieplein 5
2508 DH Den Haag (NL)**

(54) **PRODUCTION METHOD FOR EXOSOME COMPRISING TARGET PROTEIN, AND METHOD FOR TRANSFERRING TARGET PROTEIN INTO CYTOPLASM BY USING EXOSOME PRODUCED BY MEANS OF THE PRODUCTION METHOD**

(57) The present invention relates to a method for the mass-production of exosome comprising a target protein, a vector for preparing the exosome, exosome including a target protein prepared by the method, and a method for loading the target protein to cytosol by using the exosome prepared thereby. According to the method for preparing an exosome comprising a target protein provided by the present invention, the exosome loaded with a target protein can be produced with a high yield, so that it can be used broadly in the treatment of disease using the exosome.

Figure 5.

EP 3 293 265 A1

**Description**

**FIELD OF THE INVENTION**

The present invention relates to a method for preparing exosome comprising a target protein using a photospecific binding protein, and a method for delivering the target protein to cytosol using the exosome prepared by the method above.

**BACKGROUND OF THE INVENTION**

The human body is composed of about 200 kinds of 100 trillion cells, in which the physiological activity is regulated by the action of various proteins to maintain life.

Cells are surrounded by membranes in bilayer structure composed of phospholipids, which block the entry of foreign substances into cells. Most of the protein drugs which have developed so far cannot pass through the cell membrane to enter the cell and can act on the outside of the cell or act on a receptor on the cell membrane to deliver the signal into the cell in order to show physiological effect.

Cytosol has lots of proteins which interact with each other to regulate physiological activity. So, if only a protein drug can be delivered inside the cell, that is, inside the cytosol, the cell activity would be controlled more effectively.

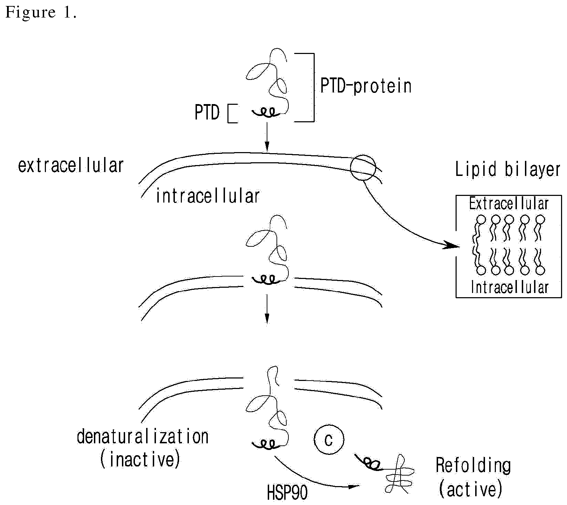

Recent studies have been actively going on to establish a method for delivering a target protein directly into cells via cell membrane. When a recombinant protein of a target protein and protein transduction domains (PTDs), the peptide that passes through the cell membrane, is prepared and administered, it can enter the cytosol through the cell membrane (Figure 1). PTD is exemplified by HIV-1 TAT, HSV VP22, Antp, dfTAT, and Hph-1. A fusion protein prepared by combining the PTDs and a target protein is produced as a recombinant protein and, at this time, a separation process is required. However, this process is problematic in that the protein refolding is not performed properly, the activity is decreased, the protein is nonspecifically transferred, the risk of causing an immune reaction *in vivo* is large, the cost is high, and the yield is low.

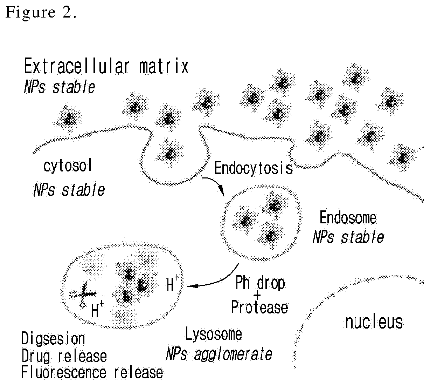

The target protein conjugated with various nanoparticles can enter the cytosol through the cell membrane by endocytosis (Figure 2). At this time, the nanoparticles are exemplified by Gold NP, Liposome NP, Magnetic NP, and Polymeric NP, etc. The separation of the nanoparticles from the target protein occurs mostly in lysosome in the cell, so the target protein is decomposed inside lysosome to lose its activity. Or the nanoparticles are difficult to be separated from the target protein in cytosol and toxicity of the nanoparticles can be another problem.

Exosome is a small vesicle with a membrane structure in the size of 50 ~ 200 nm, which is secreted out of the cell with containing protein, DNA, and RNA for intercellular signaling.

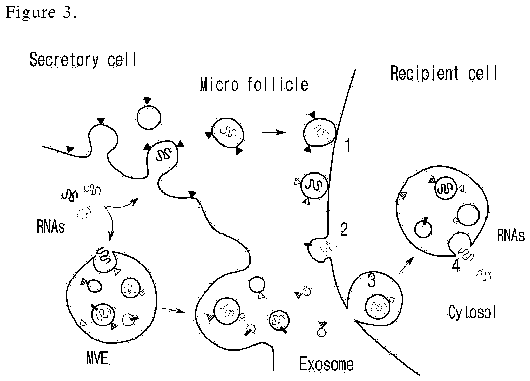

Exosome was first found in the process of leaving only hemoglobin in the red blood cells by eliminating intracellular proteins at the last stage of red blood cell maturation. From the observation under electron microscope, it was confirmed that exosome is not separated directly from plasma membrane but discharged to the extracellular space from the intracellular compartment, called multivesicular bodies (MVBs). That is, when MVBs are fused with plasma membrane, such vesicles are discharged to the outside of the cell, which are called exosome (Figure 3).

Even though the molecular mechanism of the exosome generation has not been clearly disclosed, it is known that various immune cells including B-lymphocytes, T-lymphocytes, dendritic cells, megakaryocytes, and macrophages, stem cells, and tumor cells produce and secrete exosomes when they are alive.

Exosome contains various intracellular proteins, DNA, and RNA. The substances secreted out of the cells contained in these exosomes can be reintroduced into other cells by fusion or endocytosis and serve as intercellular messengers. By analyzing such substances that are secreted out of the cell as included in exosome, specific disease can be diagnosed.

Exosome also includes various types of microRNAs. A method for diagnosing a disease by detecting the presence or absence and the abundance thereof has been reported (KR 10-2010-0127768A). International Patent Publication No WO2009-015357A describes a method for predicting and diagnosing a specific disease by detecting exosome in the cancer patient originated samples (blood, saliva, tears, etc.). In particular, the exosome obtained from a patient having a specific disease (lung disease) is analyzed and the relationship between a specific microRNA and lung disease is specifically described. Studies have been still going on to establish a method to diagnose kidney disease, in addition to lung disease, by using a specific protein included in exosome.

Additionally, exosome might also include antigens. Meanwhile, in antigen presenting cells (APC), antigen peptide is loaded in MHC (major histocompatibility complex) class II molecule in the intracellular compartment having a membrane structure including MVBs. Therefore, the exosome originated therefrom also has the antigen peptide-MHC class II complex. So, exosome acts as an immunogen carrier to present antigen peptide to CD4+ T lymphocytes and thereby can induce immune response such as T lymphocyte proliferation. The molecules that are able to stimulate immune response such as MHC class I and heat-shock proteins (HSPs) are concentrated in exosome, so that exosome can be used to increase or decrease immune response for the treatment of cancer or auto-immune disease.

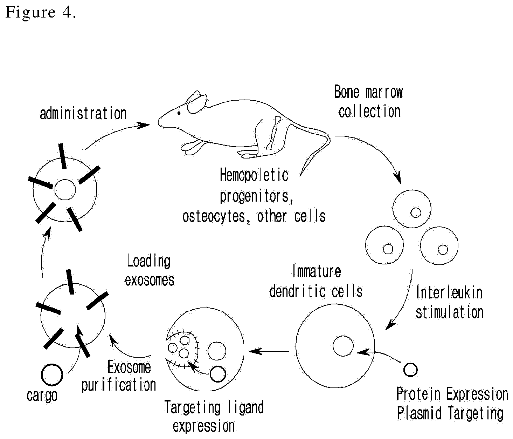

Additionally, exosome comprising a target protein can be used for the treatment of various diseases *in vivo.* For example, exosome can be prepared to include a protein or siRNA having an anticancer activity and then treated to cancer cells for cancer treatment (Figure 4).

Thus, the exosome containing a target protein can be used for the treatment of disease. To do so, exosome needs to be prepared efficiently to have a target protein. Korean Patent Publication No. 2004-0015508 describes a method for preparing exosome comprising a specific antigen. Precisely, it describes a method of discharging a target protein by using exosome, wherein a gene encoding a specific antigen is inserted in a host cell line and a protein of the introduced gene is stably expressed in the cell line which is discharged extracellularly through exosome, and a method using the exosome as a vaccine.

However, exosome is formed naturally within the cells. So, even though a gene encoding a target protein is inserted in the cell producing exosome endogenously, it is very difficult to thereby prepare exosome comprising the expressed protein in it.

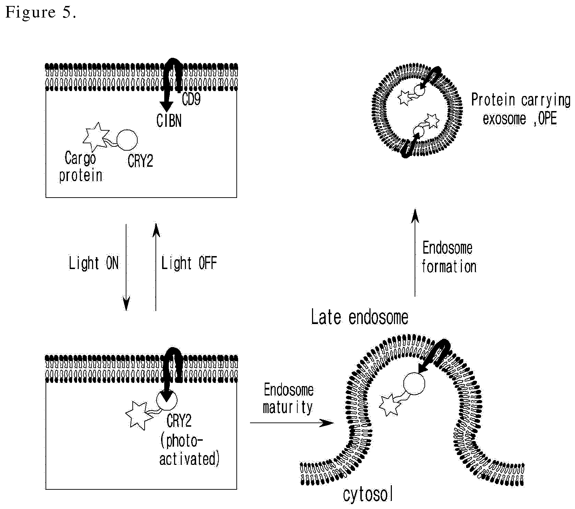

Thus, the present inventors studied to develop a method to prepare exosome comprising a target protein more efficiently. As a result, the inventors succeeded in preparing exosome comprising a target protein efficiently by expressing a fusion protein composed of an exosome specific marker and a target protein massively in the cell producing exosome endogenously at a high concentration (Figure 5).

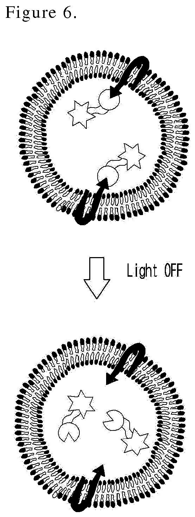

Additionally, the target protein is attached on the membrane of exosome, according to the method above. So, the fusion protein composed of a pair of an exosome specific marker and a target protein is expressed in the cell producing exosome at a high concentration, followed by irradiation to induce the linkage of the fusion protein. Then, the fusion protein is introduced inside the exosome by the action of the exosome specific marker. When the irradiation is terminated after the introduction, the fusion protein is separated into a target protein and a photo-specific binding protein inside the exosome. As a result, the exosome containing a free target protein separated from the fusion protein can be prepared efficiently (Figure 6).

The present inventors confirmed that the target protein was successfully delivered to cytosol of a target cell by using the exosome containing a target protein therein, and thereby the inventors provide a method to treat disease using exosome by regulating intracellular signaling efficiently in cytosol.

## SUMMARY OF THE INVENTION

### Technical Challenge

It is an object of the present invention to provide a method for the mass-production of exosome containing a fusion protein composed of an exosome specific marker and a target protein.

It is another object of the present invention to provide a method for the mass-production of exosome containing a target protein separated from the membrane of exosome by using a photo-specific binding protein pair.

It is also an object of the present invention to provide a vector for preparing exosome which is usable for the preparation of the exosome.

It is further an object of the present invention to provide a method to introduce a target protein in cytosol by using the exosome above.

### Technical Solution

The present invention relates to an efficient method for the mass-production of exosome containing a target protein.

The present inventors studied with various attempts to develop an efficient method for preparing exosome containing a target protein. In the course of our study, the inventors paid attention to exosome specific markers (CD9, CD63, CD81, and CD82). These markers belong to the tetraspanin family and are commonly 4-times penetration type membrane proteins. The present inventors predicted that when a target protein was conjugated on the membrane protein of exosome, the target protein would be relatively easily included in the inside of exosome.

Thus, by expressing the fusion protein composed of an exosome specific marker, which is especially rich on exosome membrane and can penetrate cell membrane, and a target protein in the cell producing exosome endogenously at a high concentration, exosome containing a target protein can be massively produced.

Particularly, the method for preparing exosome comprising a target protein of the present invention is characterized by introduction of polynucleotide encoding the fusion protein composed of an exosome specific marker and a target protein in the cell producing exosome.

At this time, in the prepared exosome, a target protein is fused with an exosome specific marker embedded in exosome membrane.

The said target protein is bound to the membrane protein of exosome and is not separated even after it arrives at the target cell. To solve this problem, various attempts have been made. As a result, a technique has been developed for the preparation of exosome comprising a target protein by conjugating a target protein temporarily to a marker protein using a photo-specific binding protein. For example, a photo-specific binding protein such as CIBN and CRY2 can be used herein. Particularly, CIBN is expressed in a form fused with CD9, which is one of the marker proteins. In the meantime, a gene encoding the fusion protein of CRY2 and a target protein is introduced in the cell producing exosome. The CIBN-CD9 fusion protein expressed in the exosome production cell can be included inside of exosome due to CD9. At this time, when the cell is irradiated with blue LED light, CRY2 domain of the target protein-CRY2 fusion protein expressed in the exosome production cell is bound to the CD9 fused CIBN domain. As a result, the reversible 'target protein-CRY2-CIBN-CD9 fusion protein' is produced. This fusion protein can be included in the inside of exosome due to CD9. Once exosome containing a target protein therein is produced and the irradiation with the blue LED light is terminated, CIBN-CRY2 link is broken and thereby the target protein remains in exosome as being apart from the cell membrane of exosome, resulting in the preparation of exosome comprising the target protein (Figures 5 ~ 10).

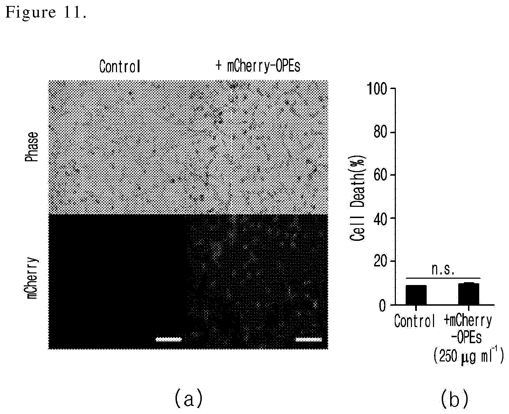

This kind of exosome prepared by the method of the present invention is completely different in its effect from the conventional exosome containing a target material. The conventional exosome is expressed as being fused onto an exosome specific marker in order to present a target protein inside of the exosome, so that the target protein, even though it is included in the inside of the exosome, is presented as being attached on the membrane of exosome, suggesting that the target protein cannot be separated from the membrane of exosome and, therefore, it can be delivered into a target cell only when the exosome is fused on the cell membrane of the target cell. Even after the exosome is fused on the target cell, the target protein remains attached on the exosome membrane and the probability that the target protein exhibits its effect in the target cell is very low. However, the exosome of the present invention presents a target protein which resides as free, and not being conjugated on the membrane of exosome. So, when such exosome enters cytosol by endocytosis of the target cell, it does not adhere to the membrane of exosome, and when the exosome is decomposed therein, the included target protein can be delivered in cytosol and is free to move in cytosol of the target cell, suggesting that the target protein is fully active with its physiological activity in the target cell cytosol (Figure 11).

In addition, the binding level of the target protein to the marker protein can be changed according to the intensity of the light to be irradiated, and by regulating the intensity of the light, the concentration of the target protein collected in exosome can be controlled.

The method for preparing exosome containing a target protein by using a photo-specific binding protein has not been reported yet and was proposed first by the present inventors.

Particularly, the method for preparing exosome containing a target protein of the present invention is composed of the following steps: (a) introducing the polynucleopeptide encoding the fusion protein (fusion protein I) composed of an exosome specific marker and the first photo-specific binding protein, and the polynucleotide encoding the fusion protein (fusion protein II) composed of a target protein and the second photo-specific binding protein that can be linked to the first photo-specific binding protein, in the exosome production cell; (b) irradiating the exosome production cell with light that can cause the conjugation between the first photo-specific binding protein and the second photo-specific binding protein; and (c) terminating the irradiation after the production of exosome has finished in the exosome production cell.

The term "exosome" in the present invention indicates a small vesicle with the plasma membrane structure, which is originated from an intracellular specific compartment called multivesicular bodies (MVBs) and is released or secreted from the cell.

In this invention, exosome plays a role as a carrier to deliver a target protein into a target cell or tissue by carrying the target protein in itself. At this time, the target protein carried by the exosome works for the target cell or tissue to help the treatment or diagnosis of a specific disease.

The term "exosome production cell" in this invention indicates the cell that is able to produce exosome.

In this invention, the exosome production cell is not limited but is preferably exemplified by B-lymphocyte, T-lymphocyte, dendritic cell, megakaryocyte, macrophage, stem cell, and tumor cell, etc. For example, in this invention, HEK293T cell that is a kind of immortalized cell line was used as the exosome production cell.

The term "exosome specific marker" in this invention indicates a protein which is rich on the membrane of exosome.

In this invention, the exosome specific marker is not limited but is preferably exemplified by CD9, CD63, CD81, and CD82, etc. For example, in a preferred embodiment of the present invention, CD9 was used as the exosome specific marker. CD9, CD63, Cd81, and CD82 are 4-times penetration type membrane proteins that allow the target protein to be easily present in exosome when the target protein is bound to the membrane protein of the exosome.

The term "photo-specific binding protein" in this invention is also called photo-induced heterodimer formation protein or photo-induced homodimer formation protein, which indicates a protein that is able to form a heterodimer by combining with different proteins or to form a homodimer by combining with another protein in the same kind when the light of a specific wavelength is irradiated.

In this invention, the photo-specific binding protein is not limited but is preferably exemplified by the photo-induced heterodimer formation protein or CIB (cryptochrome-interacting basic-helix-loop-helix protein), CIBN (N-terminal domain of CIB), PhyB (phytochrome B), PIF (phytochrome interacting factor), FKF1 (Flavinbinding, Kelch repeat, F-box 1), GIGANTEA, CRY (cryptochrome), and PHR (phytolyase homologous region), etc.

In particular, when the photo-specific binding protein is the photo-induced heterodimer formation protein, two types of photo-specific binding protein (the first and the second photo-specific binding proteins) can be used. When the first photo-specific binding protein is CIB or CIBN, the second photo-specific binding protein can be CRY or PHR. When the first photo-specific binding protein is PhyB, the second photo-specific binding protein can be PIF. When the first photo-specific binding protein is GIGANTEA, the second photo-specific binding protein can be FKF1.

For example, in a preferred embodiment of the present invention, CIBN was used as the first photo-specific binding protein, and CRY2 was used as the second photo-specific binding protein. The wavelength of the light used herein was the blue light with 460 ~ 490 nm. The intensity of the light was 20 ~ 50 $\mu$W.

In the meantime, in order to confirm the expression and to find out the location of the first fusion protein composed of the exosome specific marker and the first photo-specific binding protein expressed therein, a marker protein can be fused thereto. For example, in a preferred embodiment of the invention, the fluorescent protein EGFP was inserted in the first fusion protein wherein CIBN and CD9 or GIGANTEA and CD are linked together. So, the expression pattern (expression and expression level) and the intracellular location of the first fusion protein can be investigated by the expression of the first fusion protein as harboring the fluorescent protein EGFP.

The term "target protein" in this invention indicates a protein which is expressed as a fusion protein conjugated with the second photo-specific binding protein to locate the target protein inside the exosome.

In this invention, the target protein can be carried by exosome after being expressed in cells. The target protein is not limited but is preferably a disease treating protein or disease diagnosing protein. For example, in a preferred embodiment of the present invention, mCherry with fluorescence was used as the target protein.

The term "culture" in this invention indicates a method to grow cells or microorganisms in a properly controlled environment.

In this invention, a transformant was cultured for 1 ~ 3 days and then the medium was replaced with a fetal bovine serum-free medium, followed by further culture for 2 ~ 5 days.

In this invention, the method for culturing the transformant is any of those well known to those in the art.

The said medium herein indicates a notified medium widely used for animal cell culture, which can be selected from the group consisting of commercially available serum-free media, protein-free media, and chemically defined media.

The serum-free media above are used for animal cell culture, which are free from fetal bovine serum and are exemplified by SFM4CHO (HyClone) and EX-Cell (JHR Bioscience). Insulin like growth factor I (IGF-I), ethanolamine, ferric chloride, and phosphatidyl choline can be added to the media, but not always limited thereto.

The protein-free media above are animal cell culture media where, from the fetal bovine serum-free media, animal originated protein,s especially high molecular proteins, in particular having the molecular weight of at least 10 kDa, are eliminated. The protein-free media can be ProCHO (Lonza) and PF-CHO (HyClone), but not always limited thereto.

The chemically defined media above are animal cell culture media which do not include any animal originated components and instead have components all having defined chemical structures. The chemical defined media can be CDM4CHO (HyClone), PowerCHO2CD (Lonza), and CD-optiCHO (Life Technologies), but not always limited thereto.

The term "the first fusion protein" in this invention indicates the fusion protein made by binding between the exosome specific marker and the first photo-specific binding protein.

In this invention, the order of arrangement of the exosome specific marker and the first photo-specific binding protein contained in the first fusion protein is not limited, as long as the first photo-specific binding protein is located in the direction toward the inside of exosome when the first fusion protein is expressed in the exosome production cell. For example, N-terminal of the first photo-specific binding protein can be conjugated to C-terminal of the exosome specific marker.

Additionally, the exosome specific marker and the first photo-specific binding protein which compose the first fusion protein are linked directly to each other or can be connected by a linker. The linker above is not limited, as long as the first fusion protein is expressed in the exosome production cell with presenting the first photo-specific binding protein located in the direction toward the inside of exosome, but is preferably a peptide linker composed of amino acids and, more preferably, a flexible peptide linker. The peptide linker can be expressed by using an expression vector wherein the nucleic acids encoding the linker are connected with other nucleic acids encoding each domain in frame.

The term "the second fusion protein" indicates a fusion protein in which the second photo-specific binding protein and the target protein are combined.

In this invention, the order of arrangement of the second photo-specific binding protein and the target protein contained in the second fusion protein is not limited, as long as the second fusion protein is located inside of exosome as being conjugated with the first photo-specific binding protein region of the first fusion protein in the exosome production cell. For example, N-terminal of the target protein can be conjugated to C-terminal of the second photo-specific binding protein.

In addition, the second photo-specific binding protein and the target protein which compose the second fusion protein are linked directly to each other or can be connected by a linker. The linker above is not limited, as long as the second fusion protein is located inside of exosome as being conjugated with the first photo-specific binding protein of the first fusion protein in the exosome production cell, but is preferably a peptide linker composed of amino acids and, more preferably, a flexible peptide linker. The peptide linker can be expressed by using an expression vector wherein the nucleic acids encoding the linker are connected with other nucleic acids encoding each domain in frame.

In addition, each fusion protein above can include a polypeptide having the sequence wherein one or more amino acid residues are different from those in the wild type amino acid sequence of each domain included therein. Amino acid exchange in proteins and polypeptides without changing the overall activity of a molecule is well known to those in the art. The most common exchange occurs between Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In addition, a protein having increased structural stability against heat or pH or increased protein activity due to mutation or modification of amino acid sequence can be included.

Lastly, the fusion protein above or the polypeptide of each domain comprising the fusion protein can be prepared by the chemical peptide synthesis method well informed to those in the art, or prepared by the following method. A gene encoding each domain is amplified by PCR (polymerase chain reaction) or synthesized by the conventional method well known to those in the art. The gene is cloned in an expression vector and expressed.

In the meantime, each fusion protein can be expressed in the exosome production cell by introducing a polynucleotide encoding each fusion protein in the exosome production cell. At this time, the polynucleotide is introduced in the exosome production cell by the conventional method well informed to those in the art. For example, an expression vector can be used for the introduction.

The term "expression vector" in this invention is a recombinant vector capable of expressing a target peptide in host cells. This vector indicates a gene construct containing essential regulators operably linked so as to express the gene insert. The expression vector includes expression control elements such as a start codon, a termination codon, a promoter, and an operator. The start codon and termination codon are generally understood as a part of the nucleotide sequence encoding a polypeptide. They are supposed to be working when a gene construct is introduced and to reside in a coding sequence in frame. The promoter of the vector can be constitutive or inductive.

The term "operably linked" in this invention indicates the status when the nucleic acid expression regulation sequence functioning as usual and the nucleic acid sequence encoding a target protein or RNA are linked by functional linkage. For example, a promoter is operably linked to a nucleic acid sequence encoding a protein or RNA in order to affect the expression of the coding sequence. The functional linkage with an expression vector can be achieved by the recombinant DNA technology well known to those in the art, and particularly the site-specific DNA cleavage and linkage can be achieved by using the conventional enzyme well known to those in the art.

In addition, the said expression vector can include a signal sequence for the discharge of a fusion polypeptide in order to promote the separation of a protein from the cell culture medium. A specific initiation signal might be necessary for the efficient translation of the inserted nucleic acid sequence. These signals contain ATG start codon and its neighboring sequences. In some cases, an exogenous translational control signal, which may include the ATG start codon, should be provided. These exogenous translational control signals and start codon can be various natural and synthetic sources. The expression efficiency can be increased by the introduction of appropriate transcription or translation enhancers.

In a preferred embodiment of the present invention, the expression vector is able to express a target protein conjugated with a tag in order to confirm the insertion of a target protein inside the exosome. The tag herein is to confirm the presence of a target protein, which can be conjugated to the region opposite to the region of the second photo-specific binding protein conjugation. For example, a fluorescent protein such as a red fluorescent protein and a green fluorescent protein is used as a tag to be conjugated to C-terminal of a target protein.

The purpose for the target protein prepared as described above is expressed in the exosome production cell. Once exosome is produced, it is investigated whether or not the fluorescent protein tag is detected, by which the presence of the target protein in exosome can be confirmed.

The term "light" in this invention indicates the light to be irradiated in order to combine temporarily the first photo-specific binding protein and the second photo-specific binding protein expressed in the exosome production cell.

As described hereinbefore, the first photo-specific binding protein is expressed as the first fusion protein conjugated with the exosome specific marker, while the second photo-specific binding protein is expressed as the second fusion protein conjugated with the target protein. When the light is irradiated to the exosome production cell, the first photo-specific binding protein is combined with the second photo-specific binding protein, and as a result the fusion protein complex comprising the exosome specific marker-the first photo-specific binding protein-the second photo-specific binding protein-the target protein is formed temporarily. When exosome is produced in the exosome production cell, the target protein can be linked to the exosome due to the exosome specific marker. At this time, the target protein presents inside the exosome and when the irradiation with the light is stopped after the production of the exosome, the first photo-specific binding protein is separated from the second photo-specific binding protein and, thereby, the target protein included in the exosome is to be discharged together with the exosome as being a part of the exosome. It is preferred for the light to be irradiated to the cell intermittently rather than continually in order to deliver the target protein inside the exosome more efficiently. That is, when the light is irradiated intermittently, the conjugation and separation of the first photo-specific binding protein and the second photo-specific binding protein repeat so that the probability that the target protein is introduced into the exosome can be increased.

In the meantime, the wavelength of the light enough to induce the binding of the first photo-specific binding protein with the second photo-specific binding protein varies from the kinds of the first and the second photo-specific binding proteins. The wavelength of the light that induces the binding of the first photo-specific binding protein and the second photo-specific binding protein depends on the type of the proteins. So, the proper wavelength of the light can be selected as known to those in the art. For example, in order to link CRY2 to CIBN, the light with the wavelength of 460 ~ 490 nm is preferred. If the light is irradiated less than 10 minutes, CRY2 and CIBN are separated from each other. When PhyB is combined with PIF, the light with the wavelength of 650 nm is irradiated for 10 minutes. When the light with the wavelength of 750 nm is irradiated for 5 minutes, PhyB and PIF are separated from each other. When FKF1 is combined with GIGANTEA, the light with the wavelength of 460 nm is irradiated for 30 minutes. In a preferred embodiment of the present invention, in order to induce the binding of CIBN and CRY2, the light with the wavelength of 460 ~ 490 nm was irradiated.

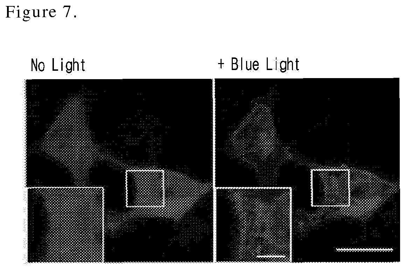

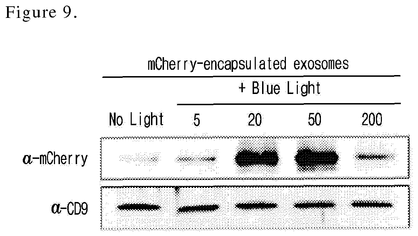

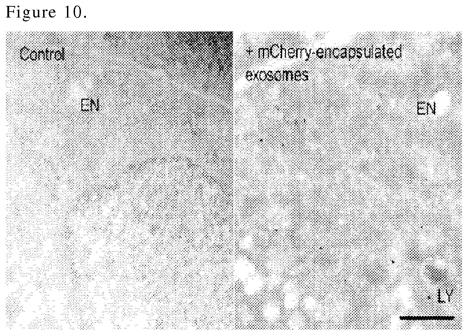

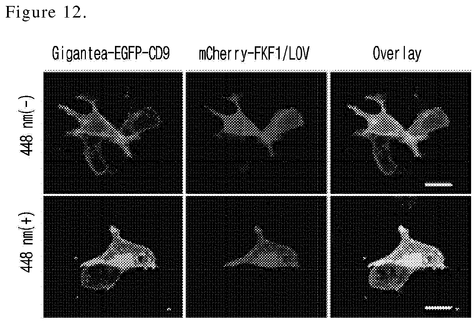

In a preferred embodiment of the present invention, the CRY2/mCherry fusion protein and the CIB/CD9 fusion protein were expressed in HEK293T, the immortalized cell line producing a large amount of exosome. As a result, the distribution of mCherry protein uniformly distributed in cytosol was found to be in cell membrane and endosome-like structure membrane when the blue light was irradiated (Figure 7). Similar results were observed when the FKF1/mCherry fusion protein and the GIGANTEA/CD9 fusion protein were expressed in HEK293T cells (Figure 12). The CRY2/mCherry fusion protein and the CIBN/CD9 fusion protein were expressed in HEK293T cells, followed by irradiation with the blue light with regulating the intensity of the light. As a result, when the light was irradiated with the intensity of 20 ~ 50 μW, the level of mCherry protein collected in exosome was the highest (Figure 9). The exosomes isolated from the cells were treated to HT1080 cells at the concentration of approximately 250 μg/mℓ. As a result, the exosomes did not show any specific cytotoxicity against the HT1080 cells and it was confirmed that the mCherry protein was delivered in the cytosol thereof (Figure 10).

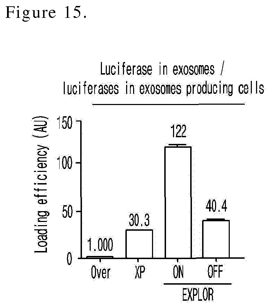

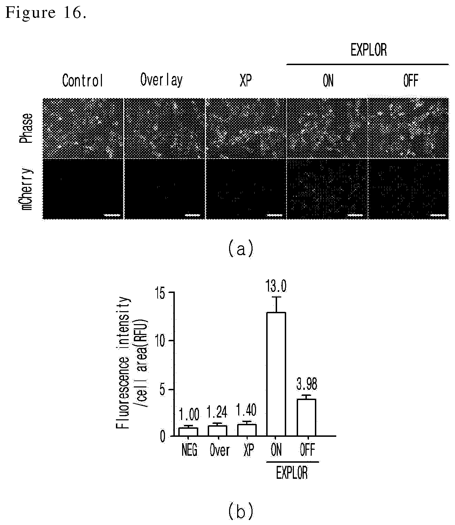

In addition, to compare the efficiency of introducing the target protein in exosome and the efficiency of exosome transfer to the target cell with those of the conventional methods, XPACK vector was used for the conventional method and the expression vectors of the CRY2/mCherry fusion protein and the CIBN/CD9 fusion protein were introduced in HEK293T cells. Then, the production of the target protein in exosome was compared. As a result, it was confirmed that the introduction efficiency was remarkably high when the method of the present invention was used (Figure 15). The exosome separated from the exosome production cell was treated to the target cell (HeLa) to compare the expression of the target protein. When the exosome separated by the method of the present invention was used, the expression of the target protein was the highest in the target cell (Figure 16).

In another preferred embodiment of the present invention, the present invention provides a vector for the production of exosome comprising (a) the first expression vector containing the polynucleotide encoding the fusion protein of the exosome specific marker and the first photo-specific binding protein (the first fusion protein); and (b) the second expression vector containing the multicloning site to which the polynucleotide encoding the target protein can be introduced and the polynucleotide encoding the second photo-specific binding protein to be linked to the first photo-specific binding protein above.

In the present invention, in the vector for the production of exosome provided by the present invention, the exosome specific marker, the first photo-specific binding protein, the exosome production cell, and the second photo-specific binding protein are the same as described above.

The term "transformed cells for exosome production" in this invention indicates the cells capable of producing exosome by expressing the first fusion protein wherein the polynucleotide encoding the fusion protein (the first fusion protein) of the exosome specific marker and the first photo-specific binding protein is introduced.

In this invention, the second expression vector includes a polynucleotide encoding the second photo-specific binding protein and a neighboring multicloning site. When a polynucleotide encoding a target protein is inserted in the multicloning site, it is expressed as the fusion protein comprising the second photo-specific binding protein and the target protein (the second fusion protein).

The vector for preparing exosome provided by the present invention can contain one or more kinds of constituents, solutions, or devices usable not only for the transformed cells for exosome production and the expression vector; but also for the introduction of the expression vector; for the culture of the transformed cells for exosome production; and for the separation and purification of the exosome produced from the transformed cells for exosome production. For example, a buffer proper for the introduction of the expression vector and a medium and a vessel necessary for the culture of the transformed cells for exosome production can be additionally included.

In another aspect of the present invention provides the exosome prepared by the method of the invention in which a target protein is included.

The exosome prepared by the method above contains a fusion protein (the first fusion protein) composed of an exosome specific marker and the first photo-specific binding protein on the plasma membrane thereof and another fusion protein (the second fusion protein) composed of the second photo-specific binding protein that can be conjugated to the first photo-specific binding protein and a target protein. So, when such exosome is treated to the target tissue cells, the second fusion protein included in the exosome can be delivered to cytosol of the target tissue cells through the fusion of the plasma membrane.

The said exosome containing a target protein can be used for the treatment of various diseases *in vivo.* For example, exosome containing a protein polymer (for example, antibody, etc.) showing the anticancer activity as a target protein is prepared, which is then treated to cancer cells. That is, the exosome can be used as a biocompatible anticancer agent better acting than the conventional liposome.

## ADVANTAGEOUS EFFECT

In the present invention, a target protein of the invention, exosome comprising a target protein can be prepared with a high yield. Also, a target protein presents as being separated from the membrane of exosome, so that it can be widely applied to treat disease.

## BRIEF DESCRIPTION OF THE DRAWINGS

The application of the preferred embodiments of the present invention is best understood with reference to the accompanying drawings, wherein:

Figure 1 is a diagram illustrating the method for delivering a target protein through a recombinant protein of a target protein and protein transduction domains (PTDs) (Steven R. et al. Protein transduction: unrestricted delivery into all cells? Trends in Cell Biology, 2000).

Figure 2 is a diagram illustrating the method for delivering a target protein to cytosol using a complex of nanoparticles and a target protein via endocytosis (Munish Chanana et al. Physicochemical properties of protein-coated gold nanoparticles in biological fluids and cells before and after proteolytic digestion. Angew. Chem. Int. Ed. 2013).

Figure 3 is a diagram illustrating the process in which exosome is separated and released from multi-vesicular bodies (MVBs) (Graca Raposo and Willem Stoorvogel. Extracellular vesicles: Exosomes, microvesicles, and friends. Cell Biology 200(4), 373-383, 2013).

Figure 4 is a diagram illustrating the process of treating cancer by delivering siRNA *in vivo* through the targeted exosome (Alvarez-Erviti, L. et al. Delivery of siRNA to the mouse brain by systemic injection of targeted exosomes. Nature Biotechnology 29, 341-345, 2011).

Figure 5 is a schematic diagram illustrating the preparation process of optogenetically-designed protein-carrying exosomes (EXPLORs) according to the present invention.

Figure 6 is a diagram illustrating the process of separating the fusion protein of a target protein and a photo-specific binding protein in the inside of exosome when the light irradiation on EXPLORs is stopped.

Figure 7 is a fluorescence image illustrating the changes in the intracellular location of mCherry protein according to the blue light irradiation in the transformed HEK293T cells introduced with CIBN-EGFP-CD9 gene and mCherry-CRY2 gene.

Figure 8 is a diagram illustrating the experimental procedure of obtaining EXPLORs according to the present invention.

Figure 9 is an immunoblot analysis image showing the results of measuring the changes of the content of a target protein (mCherry protein) captured in exosome according to the intensity of blue light.

Figure 10 is an electron micrograph illustrating the results of investigation of the introduction of a target protein in target cells after treating the target cells (HT1080) with exosome containing the target protein (mCherry), wherein the left indicates the target cells not-treated with exosome and the right indicates the target cells treated with exosome.

Figure 11 is a set of a fluorescence image (a) illustrating the results of investigation of the introduction of a target protein in target cells after treating the target cells (HT1080) with exosome containing the target protein (mCherry); and a graph (b) illustrating the results of comparison of the ratio of apoptotic cells induced by the treatment of exosome.

Figure 12 is a fluorescence image illustrating the changes in the intracellular location of mCherry protein according to the blue light irradiation in the transformed HEK293T cells introduced with GIGANTEA-EGFP-CD9 gene and mCherry-FKF1LOV.

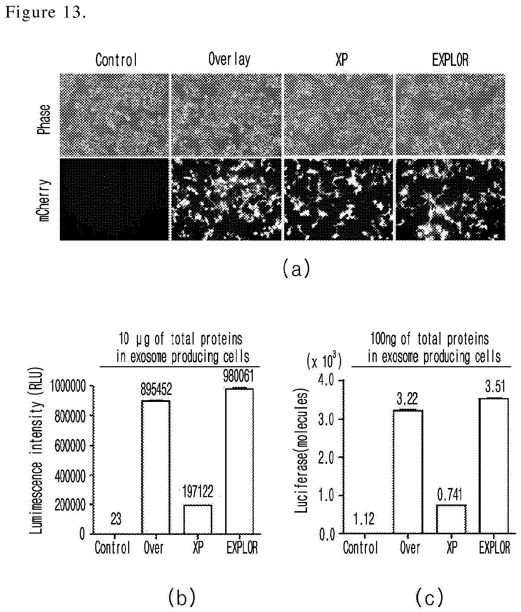

Figure 13 is a set of diagrams illustrating the expression of the Luciferase-mCherry fusion protein measured by fluorescence imaging (a) and the luciferase activity and the number of molecules in the production cells (b):

Control: HEK293T cells treated with nothing;
OVER: HEK293T cells introduced with Luciferase-mCherry-CRY2 alone;
XP: HEK293T cells introduced with XPACK-Luciferase-mCherry by using XPACK (Systems Biosciences), the commercial vector designed for exosome loading technique;
EXPLOR: HEK293T cells introduced with Luciferase-mCherry-CRY2 and CIBN-EGFP-CD9 according to the present invention.

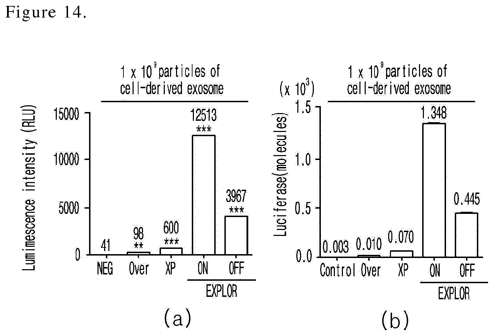

Figure 14 is a diagram illustrating the luciferase activity (a) and the number of molecules (b) in the produced exosome:

NEG: exosome produced in the HEK293T cells treated with nothing;
OVER: exosome produced in the HEK293T cells introduced with Luciferase-mCherry-CRY2;
XP: exosome produced in the HEK293T cells introduced with XPACK-Luciferase-mCherry by using XPACK (Systems Biosciences), the commercial vector designed for exosome loading technique;
EXPLOR: exosome produced in the HEK293T cells introduced with Luciferase-mCherry-CRY2 and CIBN-EGFP-CD9 according to the present invention;
ON: exosome produced by culturing under the irradiation of 200 $\mu$W blue light for 72 hours,
OFF: exosome produced by culturing under the light-free condition for 72 hours.

Figure 15 is a diagram illustrating the loading efficiency of a target protein in the exosome produced above.
Figure 16 is a diagram illustrating the transfer efficiency of a target protein into the target cells (HeLa) using exosome:

Control: exosome produced in the HEK293T cells treated with nothing;
OVER: exosome produced in the HEK293T cells introduced with Luciferase-mCherry-CRY2;
XP: exosome produced in the HEK293T cells introduced with XPACK-Luciferase-mCherry by using XPACK (Systems Biosciences), the commercial vector designed for exosome loading technique;
EXPLOR: exosome produced in the HEK293T cells introduced with Luciferase-mCherry-CRY2 and CIBN-EGFP-CD9 according to the present invention;
ON: exosome produced by culturing under the irradiation of 200 $\mu$W blue light for 72 hours,
OFF: exosome produced by culturing under the light-free condition for 72 hours.

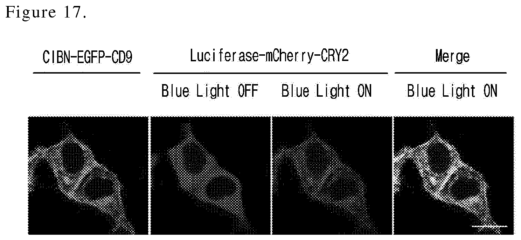

Figure 17 is a diagram illustrating the location of the expression of Luciferase-mCherry-CRY2 and CIBN-EGFP-CD9 in HEK293T cells, indicating they share the same position for the expression.

## DESCRIPTION OF THE PREFERRED EMBODIMENTS

Practical and presently preferred embodiments of the present invention are illustrative as shown in the following Examples.

However, it will be appreciated that those skilled in the art, on consideration of this disclosure, may make modifications and improvements within the spirit and scope of the present invention.

**Example 1: Preparation of exosome**

**<1-1> Confirmation of the binding of CIBN and CRY2 for the production of exosome**

PcDNA3.1(+) vector containing CIBN-EGFP-CD9 gene and pcDNA3.1(+) vector containing mCherry-CRY2 gene were introduced into HEK293T cells, the exosome production cells, under the light-free condition, followed by culture for 24 hours. The medium was replaced with a serum-free medium, followed by additional culture for 48 hours. Upon completion of the culture, the cells were irradiated with the blue light with the wavelength of 460 ~ 490 nm. The location of red fluorescence shown in mCherry before and after the blue light irradiation was confirmed by using a confocal microscope (Figure 7).

Figure 7 is a fluorescence image illustrating the changes in the intracellular location of mCherry protein according to the blue light irradiation in the transformed HEK293T cells introduced with CIBN-EGFP-CD9 gene and mCherry-CRY2 gene. As shown in Figure 7, before the blue light irradiation that could cause the binding of the photo-specific binding proteins CIBN and CRY2, mCherry protein was evenly distributed in the cytosol. However, after the blue light irradiation, mCherry protein was concentrated in the membrane. This clustering of mCherry protein was analyzed to be caused by the binding of CIBN and CRY2, the photo-specific binding proteins.

## EP 3 293 265 A1

**<1-2> Confirmation of the binding of GIGANTEA and FKF1 for the production of exosome**

PcDNA3.1(+) vector containing GIGANTEA-EGFP-CD9 gene and pcDNA3.1(+) vector containing mCherry-FKF1LOV gene were used in this example. The intracellular Exosome was confirmed by the same manner as described in Example <1-1>. (LOV in the FKF1LOV above is an abbreviation of light-oxygen-voltage domain, which indicates the domain that binds to other proteins by light in FKF1 protein, so FKF1 and FKF1LOV are in fact the same herein).

Like Example <1-1>, as shown in Figure 12, mCherry protein was evenly distributed in the cytosol before the blue light irradiation that could cause the binding of the photo-specific binding proteins GIGANTEA and FKF1. However, after the blue light irradiation, mCherry protein was concentrated in the membrane. This clustering of mCherry protein was analyzed to be caused by the binding of GIGANTEA and FKF1, the photo-specific binding proteins.

**Example 2: Exosome production and the effect of light intensity on exosome production**

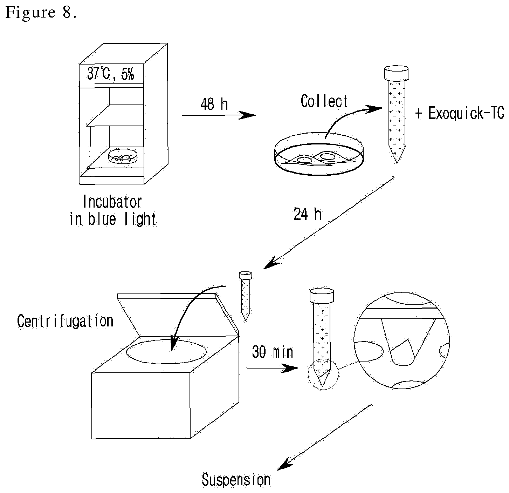

Each expression vector respectively containing CIBN-EGFP-CD9 gene and mCherry-CRY2 gene was introduced into HEK293T cells under the LED light with the wavelength of 460 nm at the intensity of 0, 5, 20, 50, and 200 $\mu$W, followed by culture for 24 hours. Then, the medium was replaced with a serum-free medium, followed by additional culture for 48 hours. Upon completion of the culture, the culture medium was separated, which was centrifuged (3000×g, 15 minutes) to obtain the supernatant excluding cell debris. ExoQuick-TC Exosome Precipitation Solution (System Biosciences, Mountain View, California, USA) was added to the obtained supernatant at the volume of 5 times the supernatant. After the mixing, centrifugation was performed (1500×g, 30 minutes) to obtain the precipitated exosome. The obtained exosome was suspended in PBS, resulting in the exosome suspension. The exosome suspension was filtered with a 0.2 $\mu$m filter using a syringe equipped with a 27-G needle. As a result, exosome in the single size was obtained (Figure 8). Then, exosome lysate was prepared by using lysis buffer, followed by immune-blotting to compare the amount of mCherry protein in the exosome (Figure 9).

Figure 9 is an immunoblot analysis image showing the results of measuring the changes of the content of a target protein (mCherry protein) captured in exosome according to the intensity of blue light. As shown in Figure 9, when the cells were irradiated with blue light at the intensity of 20 ~ 50 $\mu$W, the amount of mCherry, the target protein, in exosome was the highest. From the above results, it was confirmed that the content of the target protein captured in exosome could be regulated by controlling the intensity of the light irradiated to the cells in the course of the binding of the photo-specific binding proteins.

**Example 3: Effect of exosome treatment**

Each expression vector respectively containing CIBN-EGFP-CD9 gene and mCherry-CRY2 gene was introduced into HEK293T cells under the LED light with the wavelength of 460 nm at the intensity of 50 $\mu$W, followed by extracting exosome by the same manner as described in Example 2. The extracted exosome was treated to HT1080 cells at the concentration of 250 $\mu$g/m$\ell$ for 24 hours. The HT1080 cells were fixed on 10% gelatin gel by adding with 0.1 M phosphate buffer (pH 7.4) containing 4% PFA and 0.01% GA. The cells attached on the gelatin gel were cooled for a day by using liquid nitrogen. Thin sections cut in 45 nm by using cryoultramicrotome were obtained at -120°C. The thin sections were immuno-stained by using anti-mCherry antibody and Protein A-gold. MCherry protein was observed with Tecnai G2 Spirit Twin TEM (Figure 10).

Figure 10 is an electron micrograph illustrating the results of investigation of the introduction of a target protein in target cells after treating the target cells (HT1080) with exosome containing the target protein (mCherry), wherein the left indicates the target cells not-treated with exosome and the right indicates the target cells treated with exosome. As shown in Figure 10, it was confirmed that the target protein was transferred into the target cells when the target cells were treated with the exosome of the present invention.

**Example 4: Analysis of exosome with target protein**

Each expression vector respectively containing CIBN-EGFP-CD9 gene and mCherry-CRY2 gene was introduced into HEK293T cells under the LED light with the wavelength of 460 nm at the intensity of 50 $\mu$W, followed by extracting exosome by the same manner as described in Example 2. The extracted exosome was treated to HT1080 cells at the concentration of 250 $\mu$g/m$\ell$ for 24 hours. Then, red fluorescence was confirmed in mCherry protein under a fluorescent microscope and the ratio of dead cells was compared between the cells treated with exosome and the cells not-treated with exosome by LDH cell death assay (Figure 11).

Figure 11 is a set of a fluorescence image (a) illustrating the results of investigation of the introduction of a target protein in target cells after treating the target cells (HT1080) with exosome containing the target protein (mCherry); and a graph (b) illustrating the results of comparison of the ratio of apoptotic cells induced by the treatment of exosome.

As shown in Figure 11, it was confirmed that apoptosis did not induced by the treatment of exosome.

**Example 5: Exosome production and the comparison of introduction efficiency of a target protein into the produced exosome**

**<5-1> Confirmation of exosome production efficiency**

To compare the exosome production and the introduction efficiency of a target protein into the exosome produced thereby according to the present invention with those of the conventional method, the expression of the target protein in the exosome production cells was investigated by measuring the luciferase activity therein.

According to the conventional method, XPACK-Luciferase-mCherry was introduced in HEK293T cells by using XPACK (Systems Biosciences), the commercial vector designed for exosome loading technique (XP). On the other hand, according to the method of the invention, Luciferase-mCherry-CRY2 and CIBN-EGFP-CD9 were introduced in HEK293T cells (EXPLOR). Then, the luciferase activity in both cells was measured to compare the efficiency of the two methods. The luciferase activity was measured according to the manufacturer's instructions (Luciferase Assay Reagent, Promega). The standard curve of the results was plotted, and then the number of exosomes in the cells was quantitatively calculated.

As shown in Figure 14, it was confirmed that the method using the photo-specific binding proteins CIBN and CRY2 of the present invention was significantly higher in the introduction efficiency into exosome than the conventional method (XP) (Figure 14).

**<5-2> Expression of a target protein in the produced exosome**

The cells of Example <5-1> were cultured for 72 hours, followed by extracting exosome (Exoquick-TC, Systems biosciences). The concentration of the target protein included in the exosomes separated by the conventional method (XP) or the method of the present invention was compared indirectly by measuring the luciferase activity therein. As shown in Figure 15, it was confirmed that the method of the present invention could produce exosome containing a remarkably large amount of the target protein than the conventional method (Figure 15).

**<5-3> Comparison of the introduction efficiency of the target protein**

The introduction efficiency (E) of the target protein was calculated by the mathematical formula below based on the luciferase activity measured in Examples <5-1> and <5-2>.

[Mathematical Formula 1]

$$E = \text{measured value of luciferase activity in produced exosome} /$$

$$\text{measured value of luciferase activity in exosome production cell}$$

As shown in Figure 15, it was confirmed that the exosome produced using the binding of CRY2 and CIBN of the present invention exhibited 4 to 120 times higher efficiency than those of the other comparative groups (Figure 15).

**Example 6: Comparison of exosome transfer efficiency to target cells**

To compare the exosome transfer efficiency, the target cells were treated with exosome containing the target protein. Particularly, HeLa cells were treated with $5 \times 10^9$ exosomes for 24 hours, and then the fluorescence intensity expressed in the cells was measured. As shown in Figure 16, it was confirmed that the fluorescence intensity in the exosome of the present invention (EXPLOR) was remarkably high (Figure 16).

Therefore, it was confirmed that the method using the exosome of the present invention could deliver the target protein to the target cells more efficiently.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended Claims.

**Claims**

1. A method for the mass-production of exosome containing a target protein on its membrane, comprising the following steps:

   a) preparing transformed cells by introducing a polynucleotide encoding the fusion protein composed of an exosome specific marker and a target protein in the exosome production cells; and
   b) culturing the transformed cells of step a).

2. The method for the mass-production of exosome according to claim 1, wherein the target protein is separated from the exosome membrane and stays inside of the exosome.

3. The method for the mass-production of exosome according to claim 1 or claim 2, wherein the exosome production cell is one or more cells selected from the group consisting of B-lymphocytes, T-lymphocytes, dendritic cells, megakaryocytes, and macrophages, stem cells, and tumor cells.

4. The method for the mass-production of exosome according to claim 1, wherein the exosome specific marker is CD9, CD63, Cd81, or CD82.

5. A method for the mass-production of exosome containing a target protein, comprising the following steps:

   (a) introducing the polynucleopeptide encoding the fusion protein (fusion protein I) composed of an exosome specific marker and the first photo-specific binding protein, and the polynucleotide encoding the fusion protein (fusion protein II) composed of a target protein and the second photo-specific binding protein that can be linked to the first photo-specific binding protein, in the exosome production cell;
   (b) irradiating the exosome production cell with light that can cause the conjugation between the first photo-specific binding protein and the second photo-specific binding protein; and
   (c) terminating the irradiation after the production of exosome has finished in the exosome production cell.

6. The method for the mass-production of exosome containing a target protein according to claim 5, wherein the first photo-specific binding protein is one or more proteins selected from the group consisting of CIB (cryptochrome-interacting basichelix-loop-helix protein), CIBN (N-terminal domain of CIB), PhyB (phytochrome B), PIF (phytochrome interacting factor), FKF1 (Flavinbinding, Kelch repeat, Fbox 1), GIGANTEA, CRY (chryptochrome), and PHR (phytolyase homologous region).

7. The method for the mass-production of exosome containing a target protein according to claim 5, wherein when the first photo-specific binding protein is CIB or CIBN, the second photo-specific binding protein is CRY or PHR, and the binding of the first photo-specific binding protein and the second photo-specific binding protein is achieved by the irradiation of the light with the wavelength of 460 ~ 490 nm.

8. The method for the mass-production of exosome containing a target protein according to claim 5, wherein when the first photo-specific binding protein is PhyB, the second photo-specific binding protein is PIF, and the binding of the first photo-specific binding protein and the second photo-specific binding protein is achieved by the irradiation of the light with the wavelength of 600 ~ 650 nm.

9. The method for the mass-production of exosome containing a target protein according to claim 5, wherein when the first photo-specific binding protein is GIGANTEA, the second photo-specific binding protein is FKF1, and the binding of the first photo-specific binding protein and the second photo-specific binding protein is achieved by the irradiation of the light with the wavelength of 460 ~ 490 nm.

10. A method for delivering a protein drug to cytosol using the exosome prepared by the method of claim 1, claim 2 or claim 5.

11. A pharmaceutical composition for delivering a protein drug to cytosol containing the exosome prepared by the method of claim 1, claim 2 or claim 5 as an active ingredient.

12. A vector for the production of exosome comprising:

(a) the first expression vector containing the polynucleotide encoding the fusion protein of the exosome specific marker and the first photo-specific binding protein (the first fusion protein); and

(b) the second expression vector containing the multicloning site to which the polynucleotide encoding the target protein can be introduced and the polynucleotide encoding the second photo-specific binding protein to be linked to the first photo-specific binding protein above.

13. The vector for the production of exosome according to claim 12, wherein the second expression vector is to express the fusion protein (the second fusion protein) composed of the second photo-specific binding protein and the target protein in the exosome production cell.

14. An exosome containing a target protein prepared by the methods of claim 1, claim 2 or claim 5.

Figure 1.

Figure 2.

Extracellular matrix
*NPs stable*

cytosol
*NPs stable*

Endocytosis

Endosome
*NPs stable*

Ph drop
+
Protease

H⁺

H⁺

nucleus

Digsesion
Drug release
Fluorescence release

Lysosome
*NPs agglomerate*

Figure 3.

Figure 4.

Figure 5.

Figure 6.

Light OFF

Figure 7.

No Light    + Blue Light

Figure 8.

Figure 9.

mCherry-encapsulated exosomes

+ Blue Light

| | No Light | 5 | 20 | 50 | 200 |
|---|---|---|---|---|---|
| α-mCherry | | | | | |
| α-CD9 | | | | | |

Figure 10.

Figure 11.

(a)

(b)

Figure 12.

Gigantea-EGFP-CD9    mCherry-FKF1/LOV    Overlay

448 nm(-)

448 nm(+)

Figure 13.

| Control | Overlay | XP | EXPLOR |

Phase

mCherry

(a)

10 µg of total proteins
in exosome producing cells

980061
895452

197122

23

Control  Over   XP   EXPLOR

Lumimescence intensity (RLU)

(b)

100ng of total proteins
in exosome producing cells

(x 10³)

4.0

3.51
3.22

0.741

1.12

Control  Over   XP   EXPLOR

Luciferase(molecules)

(c)

Figure 14.

(a)

(b)

Figure 15.

Luciferase in exosomes /
luciferases in exosomes producing cells

Figure 16.

(a)

(b)

Figure 17.

| CIBN-EGFP-CD9 | Luciferase-mCherry-CRY2 | | Merge |
|---|---|---|---|
| | Blue Light OFF | Blue Light ON | Blue Light ON |

# INTERNATIONAL SEARCH REPORT

International application No.

**PCT/KR2016/004750**

| A. | CLASSIFICATION OF SUBJECT MATTER |
|---|---|

*C12N 15/85(2006.01)i, C12N 15/62(2006.01)i, C12N 5/078(2010.01)i, C12N 5/09(2010.01)i, C07K 16/28(2006.01)i, C07K 19/00(2006.01)i, A61K 47/48(2006.01)i*

According to International Patent Classification (IPC) or to both national classification and IPC

| B. | FIELDS SEARCHED |
|---|---|

Minimum documentation searched (classification system followed by classification symbols)

C12N 15/85; C12N 5/07; A61K 48/00; A61K 47/48; C12N 15/62; C12N 5/078; C12N 5/09; C07K 16/28; C07K 19/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: exosome, specific marker, target protein, light-specific coupling protein

| C. | DOCUMENTS CONSIDERED TO BE RELEVANT | |
|---|---|---|
| Category* | Citation of document, with indication, where appropriate, of the relevant passages | Relevant to claim No. |
| X | WO 2014-168548 A2 (SAMIR, El Andaloussi) 16 October 2014<br>See abstract; claims 1, 4 and 9; page 11, paragraph [3]; page 33, paragraph [2]; page 38, paragraph [2]; page 41, paragraph [3]. | 1,3-4,10-11,14 |
| A | | 2,5-9,12-13 |
| A | WO 2015-002956 A1 (OHIO STATE INNOVATION FOUNDATION) 08 January 2015<br>See abstract; claims 1, 4, 11, 24 and 36. | 1-14 |
| A | KR 10-2013-0032646 A (SAMSUNG ELECTRONICS CO., LTD.) 02 April 2013<br>See abstract; claims 1-3. | 1-14 |
| A | KOOIJMANS, Sander Aa et al., "Exosome Mimetics: a Novel Class of Drug Delivery Systems", International Journal of Nanomedicine, 2012, vol. 7, pages 1525-1541<br>See the entire document. | 1-14 |
| A | YAZAWA, Masayuki et al., "Induction of Protein-protein Interactions in Live Cells Using Light", Nature Biotechnology, (Electronic Publishing) 04 October 2009, vol. 27, no. 10, pages 941-945<br>See the entire document. | 1-14 |

☐ Further documents are listed in the continuation of Box C.      ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 05 AUGUST 2016 (05.08.2016) | **08 AUGUST 2016 (08.08.2016)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea<br>Facsimile No. 82-42-472-7140 | Telephone No. |

Form PCT/ISA/210 (second sheet) (January 2015)

INTERNATIONAL SEARCH REPORT
Information on patent family members

International application No.

**PCT/KR2016/004750**

| Patent document cited in search report | Publication date | Patent family member | Publication date |
|---|---|---|---|
| WO 2014-168548 A2 | 16/10/2014 | AU 2014-251388 A1<br>CA 2910802 A1<br>EP 2983721 A2<br>SG 11201508433 A<br>WO 2014-168548 A3<br>WO 2014-168548 A8 | 12/11/2015<br>16/10/2014<br>17/02/2016<br>27/11/2015<br>04/12/2014<br>29/01/2015 |
| WO 2015-002956 A1 | 08/01/2015 | NONE | |
| KR 10-2013-0032646 A | 02/04/2013 | US 2013-0078658 A1 | 28/03/2013 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020100127768 A
- WO 2009015357 A

- KR 20040015508

### Non-patent literature cited in the description

- **STEVEN R. et al.** Protein transduction: unrestricted delivery into all cells?. *Trends in Cell Biology,* 2000
- Physicochemical properties of protein-coated gold nanoparticles in biological fluids and cells before and after proteolytic digestion. **MUNISH CHANANA et al.** Angew. Chem. Int. 2013

- **GRACA RAPOSO ; WILLEM STOORVOGEL.** Extracellular vesicles: Exosomes, microvesicles, and friends. *Cell Biology,* 2013, vol. 200 (4), 373-383
- **ALVAREZ-ERVITI, L. et al.** Delivery of siRNA to the mouse brain by systemic injection of targeted exosomes. *Nature Biotechnology,* 2011, vol. 29, 341-345